(19) Europäisches Patentamt
European Patent Office
Office européen des brevets

(11) **EP 2 608 152 B1**

(12) **EUROPEAN PATENT SPECIFICATION**

(45) Date of publication and mention
of the grant of the patent:
**19.02.2020 Bulletin 2020/08**

(51) Int Cl.:
***G06T 7/00*** *(2017.01)*

(21) Application number: **12198518.8**

(22) Date of filing: **20.12.2012**

(54) **Medical imaging diagnosis apparatus and medical imaging diagnosis method for providing diagnostic basis**

Medizinische Abbildungsvorrichtung und medizinisches Abbildungsdiagnoseverfahren zum Bereitstellen einer diagnostischen Grundlage

Appareil de diagnostic d'imagerie médicale et procédé de diagnostic d'imagerie médicale pour fournir une base de diagnostic

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2011 KR 20110138652**

(43) Date of publication of application:
**26.06.2013 Bulletin 2013/26**

(73) Proprietor: **Samsung Electronics Co., Ltd.
Gyeonggi-do 443-742 (KR)**

(72) Inventor: **Park, Jin-Man
Gyeonggi-do (KR)**

(74) Representative: **Arnold & Siedsma
Bezuidenhoutseweg 57
2594 AC The Hague (NL)**

(56) References cited:
**WO-A2-99/05503          US-A1- 2006 274 928
US-A1- 2007 255 512**

• **IYATOMI H ET AL: "An improved Internet-based melanoma screening system with dermatologist-like tumor area extraction algorithm", COMPUTERIZED MEDICAL IMAGING AND GRAPHICS, PERGAMON PRESS, NEW YORK, NY, US, vol. 32, no. 7, 1 October 2008 (2008-10-01), pages 566-579, XP026422562, ISSN: 0895-6111 [retrieved on 2008-08-15]**

**Description**

**BACKGROUND**

**1. Field**

The following description relates to a technique of analyzing a medical image for medical diagnosis.

**2. Description of Related Art**

A computer aided diagnosis (CAD) system analyzes a medical image such as an ultrasonic image and is helpful for a doctor to make a diagnosis by displaying an abnormal area on a medical image according to the analyzed results. This CAD system has advantages in that it may reduce the uncertainty of diagnosis which inevitably occurs due to the limits of human ability to discriminate and may decrease the doctor's workload by virtue of the individual image analysis.

Typically, the CAD system provides users with the characteristic elements of an area suspected to be infected in a medical image and the diagnosis results for the infected area. For example, a display screen of the CAD system can display the characteristic elements of a lesion and whether the lesion is benign or malignant. The diagnosis results displayed on the display may correspond to the result in which the CAD system detects a particular area in the medical image and analyzes the detected area according to predetermined criteria.

Since this analyzing procedure is an algorithm performed within the CAD system, the user will not know which characteristic information of a lesion the CAD system has used to derive the diagnosis results. This reporting of diagnostic results is too simple to assure the reliability of the diagnostic results. An exemplary prior art system is known from US-2007/255512, relative to which features in the characterizing portion of the independent claims are novel and impart an inventive step on the present disclosure.

**SUMMARY**

In one general aspect, there is provided a medical imaging diagnosis apparatus exhibiting features in the appended independent apparatus claim.

In another general aspect, there is provided a medical imaging diagnosis method exhibiting features in the appended independent method claim.

Other features and aspects will be apparent from the following detailed description, the drawings, and the claims.

**BRIEF DESCRIPTION OF THE DRAWINGS**

FIG. 1 is a diagram illustrating an example of a diagnosis rule in accordance with a general aspect.
FIG. 2 is a diagram illustrating an example of a medical imaging diagnosis apparatus in accordance with a general aspect.
FIG. 3 is a diagram illustrating examples of diagnosis rules classified depending on the degree of importance in accordance with a general aspect.
FIG. 4 is a diagram illustrating an example of a method of displaying diagnostic results in accordance with a general aspect.
FIG. 5 is a diagram illustrating an example of a method of displaying diagnostic results in accordance with another general aspect.
FIG. 6 is a diagram illustrating an example of a method of displaying diagnostic results in accordance with yet another general aspect.
FIG. 7 is a diagram illustrating an example of a method of displaying diagnostic results in accordance with still another general aspect.
FIGS. 8A, 8B, and 8C illustrate an example of a method of updating diagnostic results in accordance with a general aspect.
FIG. 9 is a diagram illustrating an example of an imaging method for medical diagnosis in accordance with a general aspect.

Throughout the drawings and the detailed description, unless otherwise described, the same drawing reference numerals will be understood to refer to the same elements, features, and structures. The relative size and depiction of these elements may be exaggerated for clarity, illustration, and convenience.

## DETAILED DESCRIPTION

The following description is provided to assist the reader in gaining a comprehensive understanding of the methods, apparatuses, and/or systems described herein. Accordingly, various changes, modifications, apparatuses, and/or systems described herein will be suggested to those of ordinary skill in the art. In addition, descriptions of well-known functions and constructions may be omitted for increased clarity and conciseness.

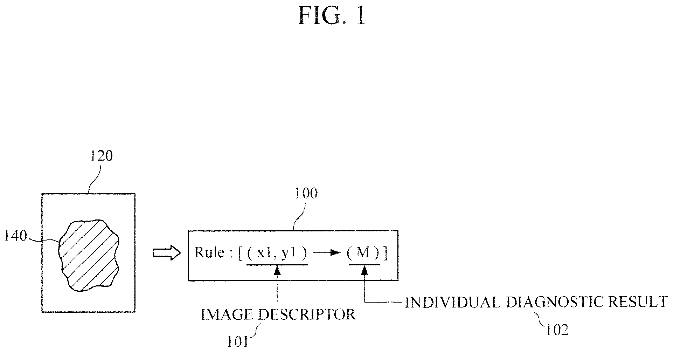

FIG. 1 is a diagram illustrating an example of a diagnosis rule 100 in accordance with a general aspect. With reference to FIG. 1, a diagnosis rule 100 includes an image descriptor (or a combination of the image descriptors) 101 and an individual diagnostic result 102.

The image descriptor 101 indicates a factor that gives a description of a visual characteristic element in the image. If an image is a medical image such as an ultrasound image, the image descriptor 101 may be characteristic information of an image that is necessary for medical diagnosis. For example, if a certain medical image 120 includes a lesion (e.g., tumor) 140, then the image descriptor 101 may be a means for representing the characteristics that are related to the lesion 140, such as shape, color, texture, orientation, and darkness. For example, in FIG. 1, 'x1' of the image descriptor 101 may be an image descriptor related to a first characteristic (e.g., shape) of the lesion 140. In other words, the 'x1' may indicate a circular shape; while an image descriptor 'x2' (not shown) may indicate an elongated shape. Similarly, 'y1' of the image descriptor 101 may be an image descriptor related to a second characteristic (e.g., color) of the lesion 140. In other words, the 'y1' may indicate the color white, while image descriptor 'y2' (not shown) may indicate the color black. It should be noted that these are merely examples, and general aspects are not limited thereto.

An individual diagnostic result 102 indicates a certain result that corresponds to the image description 101 and is related to an image 120. If the image is a medical image such as an ultrasound image, the individual diagnostic result 102 may be a medical opinion regarding a lesion included in the ultrasound image. For example, as shown in FIG. 1, 'M' of the individual diagnostic result 102 may indicate that the lesion is malignant (in contrast, if the lesion is benign, it may be represented as 'B').

Thus, if a tumor 140 included in a certain ultrasound image 120 has a circular shape (e.g., x1) and is white (e.g., y1), a diagnosis rule as shown in FIG. 1 may indicate that the tumor 140 is very likely to be malignant (e.g., M).

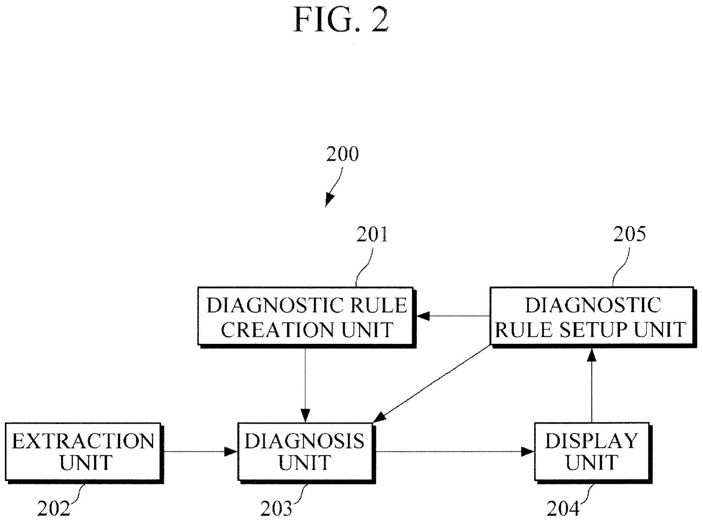

FIG. 2 is a diagram illustrating an example of a medical imaging diagnosis apparatus 200 in accordance with a general aspect. With reference to FIG. 2, a medical imaging diagnosis apparatus 200 according to a general aspect includes an extraction unit 202, a diagnosis unit 203, and a display unit 204, a diagnosis rule creation unit 201, and a diagnosis rule setup unit 205. It is noted that the elements of the medical imaging diagnosis apparatus 200 are merely examples thereof, and general aspects are not limited thereto.

The diagnosis rule creation unit 201 collects a number of reference medical images. The reference medical images may be medical images on which a medical opinion is already reflected. In other words, a certain reference medical image may include an image descriptor and an individual diagnostic result that is determined medically. The image descriptor of the certain reference medical image is a characteristic of the image that is important to consider when a doctor investigates and assesses the reference medical image.

In addition, the diagnosis rule creation unit 201 generates a number of diagnostic rules using the collected reference medical images. For example, the diagnosis rule creation unit 201 may create several diagnostic rules as shown in FIG. 1 by analyzing the image descriptor included in the collected reference medical images and the individual diagnostic results. An example of the analyzing method may employ a variety of data mining techniques.

Further, the diagnostic rule creation unit 201 assigns a predetermined degree of importance to each of the created diagnostic rules. The degree of importance may indicate a relative ranking between the diagnostic rules. When a diagnostic rule having a relatively high ranking is used, more accurate diagnostic results may be derived. This degree of importance may be defined using a variety of methods, and, in addition, may be a value modifiable by a user.

A method of assigning the degree of importance will hereby be described:

(1) Reference medical images including the image descriptor and doctor's diagnostic result are collected. The collected images, the diagnostic result, and a set of information can be represented as follows:

* Image: $I = \{i_k | 1 < k < x\}$
* Diagnostic result: $D = \{ dk | 1 < k < y\}$
* Collected set of information: $DSET = \{(i_m, d_n) | i_m \subset I, d_k \subset D\}$

(2) An image descriptor is selected. In this case, an image descriptor ID and all image descriptors included in a certain image may be represented as follows:

* Image descriptor: $ID = \{id_k | 1 < k < y, (\exists i_k \in I, id_k \text{ is in the } i_k)\}$

* All image descriptors included in a certain image: ID($i_k$)

(3) It is investigated whether there is a "pair of an image descriptor (or a combination of image descriptors) and an individual diagnostic result." If there is such a pair, the pair is selected as a diagnosis rule. In this case, the combination of image descriptors and the diagnostic rule may be represented as follows:

* Combination of image descriptors: IDComb = {idcomb$_k$|idcomb$_k$ $\in$ ID', ID'$\subset$ ID }

* Diagnostic rule: R = {$r_k$ : IDComb$_i \rightarrow$ d$_j$|($\exists i_k \in$ I, IDComb$_i \subset$ ID ($i_k$)), ($\exists$dset$_k$ ($i_k$, $r_k$) $\in$ DSET)}

(4) When the diagnostic rule is determined, support and confidence for each of the diagnostic rules are calculated as follows:

* Support (r) = Number of images that have IDComb$_i$ / Number of images
* Confidence (r) = Number of images that have IDComb$_i$ & d$_j$ / Number of images that have IDComb$_i$

(5) The diagnosis rules that satisfy a predetermined critical condition (N, M) for the support and the confidence are defined as follows:
* RefinedRule: RR = {$r_k$| $\exists r_k \in$ R, Support (R) > M, Confidence (R) > N}

(6) An accuracy of each image descriptor is calculated as follows:
* DescriptorAccuracy($id_k$) = Sensitivity ($id_k$) * v + Specificity ($id_k$) * $\mu$, where, Sensitivity ($id_k$) = Number of true positives ($id_k$)/(Number of true positives + Number of false negatives (idk)), Specificity (idk) = Number of true negatives / (Number of true negatives + number of false negatives)
The number of true positives (idk) indicates the number of times that an image descriptor determined to be present in the image is in fact present in the image. The number of false negative (idk) indicates the number of times that an image descriptor determined not to be present in the image is in fact present in the image. The number of false positives (idk) indicates the number of times that an image descriptor determined to be present in the image is not in fact present in the image. The number of true negative (idk) indicates the number of times that an image descriptor determined not to be present in the image is not in fact present.

(7) Accuracy of the diagnostic rule is given as follows:

$$* \text{ Accuracy of the diagnostic rule: RuleAccuracy ( rk )}$$

$$= \alpha * \text{Support (rk)} * \beta * \text{Confidence (rk)} * \prod \lambda k * \text{DescriptorAccuracy (id}_k)$$

The degree of importance of the diagnostic rule may be determined by the support and confidence of the diagnostic rule and the accuracy of the image descriptor composing the diagnostic rule. It should be noted that these are merely examples of the degree of importance, and the degree of importance may be defined in other ways. In addition, the degree of importance assigned to each diagnostic rule or the accuracy of the image descriptor may be updated later according to user feedback.

The extraction unit 202 receives a target medical image. The target medical image may be a medical image to be diagnosed. The type of the target medical image is not particularly limited. The target medical image may be a variety of images obtained for medical diagnosis, such as an ultrasound image or an MRI image.

Furthermore, the extraction unit 202 extracts an image descriptor from the received target medical image. For example, the extraction unit 202 may extract characteristic information related to an area suspected to be a lesion in the target medical image as an image descriptor.

The diagnosis unit 203 derives a comprehensive diagnostic result for the target medical image using the image descriptor that is extracted by the extraction unit 202 and a number of diagnostic rules. As an example, the diagnosis unit 203 may select a diagnostic rule including one or more image descriptors extracted by the extraction unit 202 among one or more diagnostic rules. The diagnostic rules may be created by the diagnostic rule creation unit 201, but are not limited thereto.

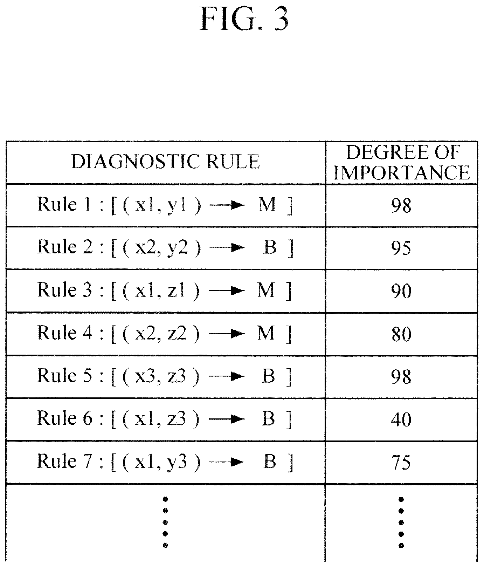

FIG. 3 is a diagram illustrating examples of diagnosis rules classified depending on the degree of importance in accordance with a general aspect.

With reference to FIG. 2 and FIG. 3, the diagnostic rule creation unit 201 may create a number of diagnostic rules from a reference medical image and assign a predetermined degree of importance to each of the created diagnostic rules. As an example, for Rule 1, when there are the image descriptor of x1 (e.g., information relating a particular shape of a lesion) and the image descriptor of y1 (e.g., information relating a particular orientation of the lesion), Rule 1 can indicate that an individual diagnostic result of M (e.g., medical opinion that the lesion is malignant) is derived. A degree of importance of 98 is thereby assigned to Rule 1.

Further, the diagnosis unit 203 may select a diagnostic rule including an image descriptor extracted from the target medical image by considering the degree of importance. As an example, it is assumed that the extracted image descriptors are "x1", "y1", and "z3". The diagnosis unit 203 may preliminarily select diagnostic rules, namely Rules 1, 3, 5, and 6 including the extracted image descriptors x1, y1, z3, or any combination thereof among a number of the created diagnostic rules.

In view of the degree of importance of the preliminarily selected diagnostic rules, a final diagnostic rule to be used in diagnosing may be selected. As an example, when a policy using two diagnostic rules having higher rank than others is set according to the degree of importance, the diagnosis unit 203 selects only Rule 1 (degree of importance of 98) and Rule 5 (degree of importance of 98). As another example, when a policy which uses only a diagnostic rule having a degree of importance of 50 or more is set, Rule 1 (degree of importance of 98), Rule 3 (degree of importance of 90), and Rule 5 (degree of importance of 98) are selected.

Referring again to FIG. 2, the diagnosis unit 203 derives a comprehensive diagnostic result for the target medical image using the selected diagnostic rule. The comprehensive diagnostic result may be a finally determined diagnostic result based on individual diagnostic results included in the selected diagnostic rules. As an example, when Rule 1, Rule 3 and Rule 5 are selected in FIG. 3, the number of the individual diagnostic results determined as malignant (M) is two and the number of the individual diagnostic results determined as benign (B) is one, so a comprehensive diagnostic result determined to be malignant may be derived. As another example, when Rule 1, Rule 3 and Rule 5 are selected in FIG. 3, malignant (M) and benign (B) may be both derived as a comprehensive diagnostic result. In this case, the comprehensive diagnostic result may be represented such that "the lesion included in the target medical image may be malignant or benign, though the lesion is more likely to be malignant than benign." As yet another example, when Rule 1 and Rule 7 are selected in FIG. 3, the individual diagnostic result of Rule 1 having a higher degree of importance is derived as the comprehensive diagnostic result. Individual diagnostic results included in the diagnostic rules selected as mentioned above are consistent with or different from one another. A method of deriving the comprehensive diagnostic result using the individual diagnostic results is not particularly limited.

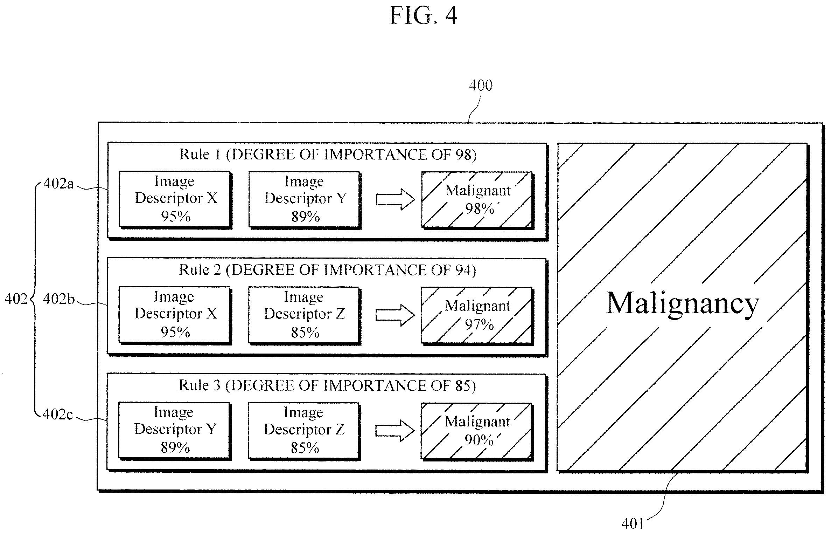

The display unit 204 provides the user with the derived comprehensive diagnostic result together with the diagnostic rule used in deriving the comprehensive diagnostic result. In other words, a certain display unit may display a diagnostic result together with a basis for deriving such diagnostic result, thereby improving the reliability of diagnosis. The display unit 204 may provide and display the diagnostic result and diagnostic basis to the user in a variety of ways. Specific examples of this will be described later with reference to FIGS. 4-7.

The diagnostic rule setup unit 205 may update a diagnostic rule based on user feedback for a comprehensive diagnosis result. For example, the diagnostic rule setup unit 205 may update the accuracy of a comprehensive diagnostic rule, the degree of importance of the diagnostic rule, the accuracy of the image descriptor included in the diagnostic rule, the accuracy of the individual diagnostic result included in the diagnostic rule, or any combination thereof. Furthermore, the diagnostic rule setup unit 205 may delete the diagnostic rule or the image descriptor included in the diagnostic rule. In addition, the diagnostic rule setup unit 205 may set a method of using the diagnostic rule of the diagnosis unit 203 and a policy for using the diagnostic rule related to the production of the comprehensive diagnostic result.

FIG. 4 is a diagram illustrating an example of a method of displaying diagnostic results in accordance with a general aspect. Referring to FIG. 2 and FIG. 4, a comprehensive diagnostic result 401 and a diagnostic basis 402 may be displayed on a diagnostic screen 400. The diagnostic screen 400 may be a variety of a monitor or a touch screen. The comprehensive diagnostic result 401 may be a medical opinion of a target medical image that is derived by the diagnosis unit 203. The diagnostic basis 402 may be diagnostic rules used to derive the comprehensive diagnostic result 401.

In accordance with a general aspect, the display unit 204 may display the comprehensive diagnostic result 401 and the diagnostic basis 402 based on a character, image, color, brightness, luminance, or any combination thereof. As an example, the display unit 204 may display the comprehensive diagnostic result 401, the image descriptor of the diagnostic basis 402, and the individual diagnostic result as a character. As another example, the display unit 204 may display each of the image descriptors as different colors.

In accordance with an additional general aspect, the display unit 204 may display the accuracy of the comprehensive diagnostic rule, the degree of importance of the diagnostic rule, the accuracy of the image descriptor included in the diagnostic rule, the accuracy of the individual diagnostic result included in the diagnostic rule by means of a character, image, color, brightness, luminance, or any combination thereof. For example, the display unit 204 may present each of the accuracy and degree of importance as a numerical value. As another example, for Rule 1 (402a) and Rule 2 (402b), because Rule 1 (402a) has a higher degree of importance than Rule 2, Rule 1 (402a) may be displayed brighter than Rule 2. As yet another example, when there is an image descriptor X and an image descriptor Y in Rule 1 (402a), a brightness of the image descriptor X may be displayed as greater than a brightness of the image descriptor Y, because the accuracy of the image descriptor X is greater than the accuracy of the image descriptor Y.

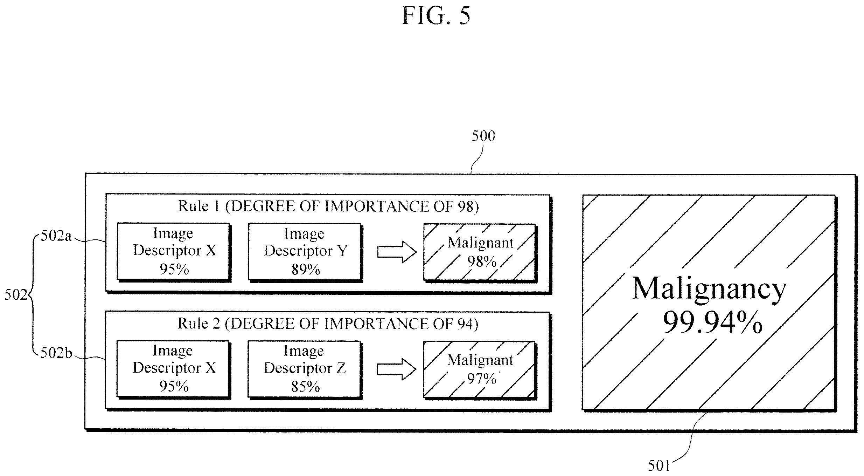

FIG. 5 is a diagram illustrating an example of a method of displaying diagnostic results in accordance with another general aspect. With reference to FIG. 2 and FIG. 5, a comprehensive diagnostic result 501 and a diagnostic basis 502 may be displayed on a diagnostic screen 500 according to another general aspect. For example, when the comprehensive diagnostic result 501 has no contradictory facts, the display unit 204 may group the diagnostic rules having the same individual diagnostic results and display the comprehensive diagnostic result by highlighting. As a further example, when the diagnostic rule selected by the diagnosis unit 203 has the same diagnostic rules such as Rule 1 (502a) and Rule 2 (502b), the display unit may display the individual diagnostic rules as the comprehensive diagnostic result 501, together with certain reliability. The reliability may be calculated a variety of ways. In this general aspect, the reliability may be calculated using the accuracy of the individual diagnostic rule (i.e., 98% and 97%).

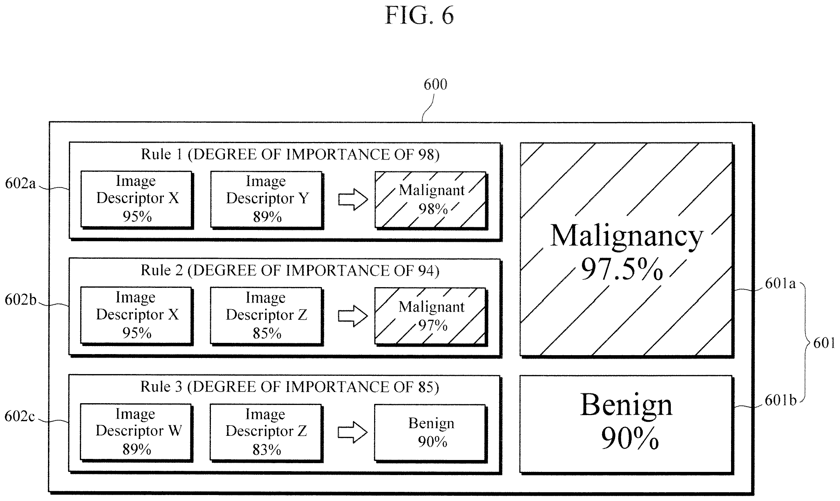

FIG. 6 is a diagram illustrating an example of a method of displaying diagnostic results in accordance with another general aspect. With reference to FIG. 2 and FIG. 6, a comprehensive diagnostic result 601 and a diagnostic basis 602 can be displayed on a diagnostic screen 600 according to yet another general aspect. As an example, when the comprehensive diagnostic result 601 has contradictory facts, the display unit 204 may classify the diagnostic rules having different individual diagnostic results and display the contradictory facts individually. In other words, when the diagnostic rules selected by the diagnosis unit 203 are Rule 1 (602a), Rule 2 (602b), and Rule 3 (602c), the individual diagnostic results (i.e., malignant) of Rule 1 (602a) and Rule 2 (602b) may not be consistent with the individual diagnostic result (i.e., benign) of Rule 3 (602c). In this case, each individual diagnostic result may be classified, and then all of them may be displayed as the comprehensive diagnostic result 610. At this time, the comprehensive diagnostic result 601a that corresponds to an individual diagnostic result having a higher accuracy may be displayed by highlighting a shape or color compared to the contradictory comprehensive diagnostic result 601b.

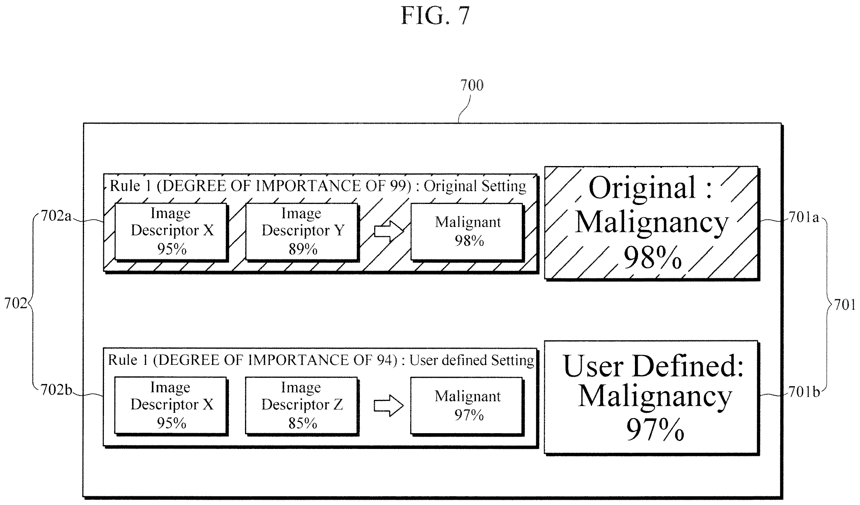

FIG. 7 is a diagram illustrating an example of a method of displaying diagnostic results in accordance with still another general aspect. With reference to FIG. 2 and FIG. 7, a comprehensive diagnostic result 701 and a diagnostic basis 702 are both displayed on a diagnostic screen 700 according to still another general aspectan embodiment. As an example, when a diagnostic rule is updated by the diagnostic rule setup unit 205, the accuracy of a comprehensive diagnostic result 701a based on a diagnostic rule before updating may be different from the accuracy of a comprehensive diagnostic result 701b based on a diagnostic rule after updating. In this case, the display unit 204 may display comprehensive diagnostic results before and after updating, together with their differences. For example, the comprehensive diagnostic result 701a before updating and its associated diagnostic rule 702a may both be displayed together. In addition, the comprehensive diagnostic result 701b after updating and its associated diagnostic rule 702b may both be displayed together.

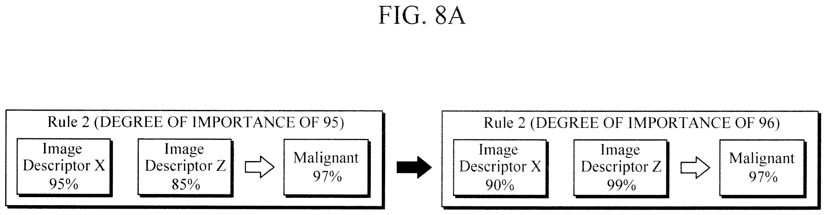

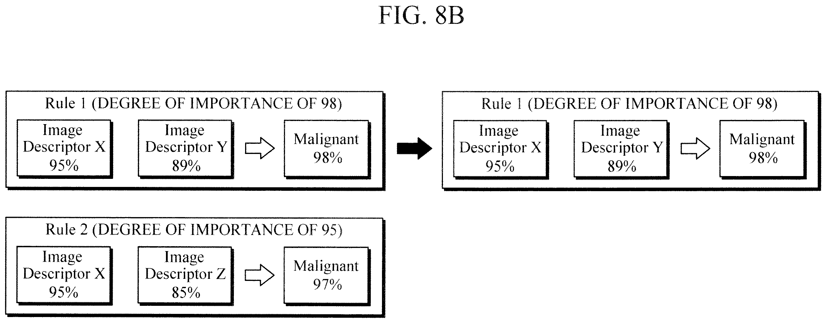

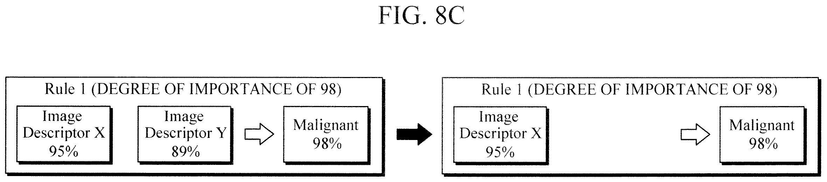

FIGS. 8A, 8B, and 8C illustrate a method of updating diagnostic results in accordance with a general aspect. With reference to FIG. 2 and FIGS. 8A to 8C, the diagnostic rule setup unit 205 may receive user feedback and edit a diagnostic rule used in diagnosis or each element included in the diagnostic rule. As an example, the diagnostic rule setup unit 205, as shown in FIG. 8A, may correct the accuracy of the diagnostic rule, the accuracy of the image descriptor, or the accuracy of the individual diagnostic result, depending on the user feedback. As another example, the diagnostic rule setup unit 205, as shown in FIG. 8B, may delete a particular diagnostic rule from the diagnostic rule setup unit 201 depending on the user feedback. As yet another example, the diagnostic rule setup unit 205, as shown in FIG. 8C, may delete an image descriptor included in a diagnostic rule depending on the user feedback.

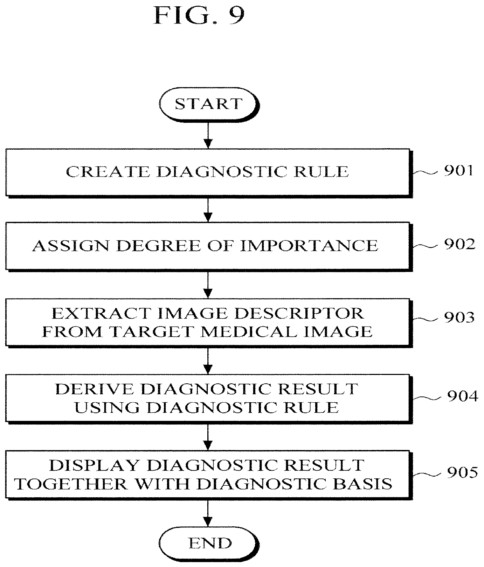

FIG. 9 is a diagram illustrating a medical imaging diagnosis method in accordance with a general aspect. This method may be implemented, for example, by apparatus shown in FIG. 2. Referring to FIG. 9, a diagnostic rule is first created (901). For example, the diagnostic rule creation unit 201 may collect a reference medical image and create a number of diagnostic rules as shown in FIG. 1.

When a diagnostic rule is created, the degree of importance is assigned to each diagnostic rule (902). For example, the diagnostic rule creation unit 201 may calculate the degree of importance of each diagnostic rule based on the support and confidence of the diagnostic rule and the accuracy of the image descriptor.

When the degree of importance is assigned to each diagnostic rule, an image descriptor is extracted from a target medical image (903). For example, the extraction unit 202 may extract characteristic information of the lesion from the target medical image.

When the image descriptor is extracted, a diagnostic result is derived based on the created diagnostic rule and the extracted image descriptor (904). For example, the diagnosis unit 203 may derive a comprehensive diagnostic result by selecting several diagnostic rules among the diagnostic rules in which the degree of importance is assigned and analyzing the individual diagnostic result of the selected diagnostic rule.

When a diagnostic result is derived, the derived diagnostic result and a diagnostic basis used in deriving the diagnostic result are both displayed (905). For example, the display unit 204 may display the comprehensive diagnostic result and the diagnostic rule in a variety of ways such as shown in FIG. 4 to FIG. 7.

If necessary, a diagnostic rule may be updated depending on user feedback (906). For example, if necessary, the diagnostic rule setup unit 205 can update the diagnostic rule or elements included in the diagnostic rule as shown in FIGS. 8A to 8C.

According to the teachings above, there is provided an apparatus and method that may provide a diagnostic result having high reliability to a user, because the diagnostic result is provided together with a related diagnostic basis. In addition, since a degree of importance is assigned to each diagnostic rule and the diagnostic rule is used in consideration of the degree of importance, it may be possible to derive the diagnostic result having high reliability.

Program instructions to perform a method described herein, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable storage media include magnetic media, such as hard disks, floppy disks, and magnetic tape; optical media such as CD ROM disks and DVDs; magnetooptical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable storage mediums. In addition, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein. In addition, the described units to perform an operation or a method may be hardware, software, or some combination of hardware and software. For example, the units may be a software package running on a computer or the computer on which that software is running.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components. Accordingly, other implementations are within the scope of the following claims.

## Claims

1. A medical imaging diagnosis apparatus (200) for providing a diagnostic basis (402, 502, 602, 702) when performing computer aided diagnosis to diagnose the presence of a lesion in a target medical image, the apparatus comprising:

   a display;
   a memory configured to store instructions therein; and
   a processor, upon execution of the instructions, configured:

   to extract characteristic information related to an area suspected to be a lesion in the target medical image, as one or more image descriptors (101) from the target medical image (120);
   to derive a comprehensive diagnostic result (401, 501, 601, 701) for the target medical image using one or more diagnostic rules (100, 402a, 402b, 402c, 502a, 502b, 602a, 602b, 602c, 702a, 702b), each of the diagnostic rules comprising:

   one or more of the extracted image descriptors; and
   an individual diagnostic result (102) corresponding to the one or more of the extracted image descriptors; and

   to control the display to display the derived comprehensive diagnostic result together with the diagnostic basis comprising the diagnostic rules and the corresponding image descriptors used in deriving the comprehensive diagnostic result.

2. The medical imaging diagnosis apparatus of claim 1, wherein the processor is further configured to control the display to display the derived comprehensive diagnostic result, the diagnostic rules, or a combination thereof based on a character, image, color, brightness, luminance, or any combination thereof; and/or wherein the processor is further configured to control the display to display accuracy of the derived comprehensive diagnostic result, a degree of importance of the diagnostic rules used in deriving the comprehensive diagnostic result, accuracy of the extracted image descriptors included in the diagnostic rules, accuracy of the individual diagnostic result included in the diagnostic rules, or any combination thereof based on a character, image, color, brightness, luminance, or any combination thereof.

3. The medical imaging diagnosis apparatus of claim 1 or 2, wherein, when the derived comprehensive diagnostic result has no contradictory facts, the processor is further configured to group the diagnostic rules having same individual diagnostic results and control the display to display the comprehensive diagnostic results by highlighting; and/or

when the derived comprehensive diagnostic result has contradictory facts, the controller is further configured to classify the diagnostic rules having different individual diagnostic results and control the display to display the contradictory facts individually.

4. The medical imaging diagnosis apparatus of one of claims 1 - 3, wherein the processor is further configured to:

create one or more of the diagnostic rules using one or more reference medical images; and
assign a predetermined degree of importance to each of the created diagnostic rules; and

wherein the processor is further configured to calculate the predetermined degree of importance based on a support and a confidence of each of the created diagnostic rules and an accuracy of each of the image descriptors included in the created diagnostic rules.

5. The medical imaging diagnosis apparatus of claim 4, wherein the processor is further configured to:

select one or more of the diagnostic rules from the created diagnostic rules according to the predetermined degree of importance of the created diagnostic rules; and
derive the comprehensive diagnostic result for the target medical image using the selected diagnostic rules.

6. The medical imaging diagnosis apparatus of one of claims 1 - 5, wherein the processor is further configured to update the one or more diagnostic rules based on user feedback for the derived comprehensive diagnostic result.

7. The medical imaging diagnosis apparatus of claim 6, wherein the processor is further configured to update accuracy of the derived comprehensive diagnostic result, a degree of importance of the diagnostic rules used in the deriving the comprehensive diagnostic result, accuracy of the extracted image descriptors included in the diagnostic rules, accuracy of the individual diagnostic result included in the diagnostic rules, or any combination thereof according to the user feedback.

8. The medical imaging diagnosis apparatus of claim 6 or 7, wherein the processor is further configured to delete one or more of the diagnostic rules, one or more of the extracted image descriptors included in the diagnostic rules, or a combination thereof according to the user feedback.

9. The medical imaging diagnosis apparatus of one of claims 6 - 8, wherein, when the derived comprehensive diagnostic result is different from a comprehensive diagnostic result after updating of the one or more diagnostic rules, the processor is further configured to display the derived comprehensive diagnostic result, the comprehensive diagnostic result after updating of the one or more diagnostic rules, and differences between the derived comprehensive diagnostic result and the comprehensive diagnostic result after updating of the one or more diagnostic rules.

10. A medical imaging diagnosis method for providing a diagnostic basis when performing computer aided diagnosis to diagnose the presence of a lesion in a target medical image, the method comprising:

extracting characteristic information related to an area suspected to be a lesion in the target medical image as one or more image descriptors from the target medical image;
deriving a comprehensive diagnostic result for the target medical image using one or more diagnostic rules, each of the diagnostic rules including one or more of the extracted image descriptors and an individual diagnostic result corresponding to the one or more of the extracted image descriptors; and
displaying the derived comprehensive diagnostic result together with the diagnostic basis comprising the diagnostic rules and the corresponding image descriptors used in deriving the comprehensive diagnostic result.

11. The medical imaging diagnosis method of claim 10, further comprising:

creating one or more of the diagnostic rules using one or more reference medical images; and

assigning a predetermined degree of importance to each of the created diagnostic rules.

**12.** The medical imaging diagnosis method of claim 10, further comprising updating the one or more diagnostic rules based on user feedback for the derived comprehensive diagnostic result.

**Patentansprüche**

**1.** Medizinische Abbildungsdiagnosevorrichtung (200) zum Bereitstellen einer diagnostischen Grundlage (402, 502, 602, 702) bei der Durchführung einer computergestützten Diagnose, um das Vorhandensein einer Läsion in einem medizinischen Zielbild zu diagnostizieren, wobei die Vorrichtung Folgendes umfasst:

eine Anzeige;
einen Speicher, der zum Speichern von Anweisungen darin konfiguriert ist; und
einen Prozessor, der bei der Ausführung der Anweisungen zu Folgendem konfiguriert ist:

Extrahieren von charakteristischen Informationen, die mit einem Bereich in Verbindung stehen, von dem vermutet wird, dass es sich um eine Läsion in dem medizinischen Zielbild handelt, als ein oder mehrere Bilddeskriptoren (101) aus dem medizinischen Zielbild (120);
Ableiten eines umfassenden Diagnoseergebnisses (401, 501, 601, 701) für das medizinische Zielbild unter Verwendung einer oder mehrerer Diagnoseregeln (100, 402a, 402b, 402c, 502a, 502b, 602a, 602b, 602c, 702a, 702b) abzuleiten, wobei jede der Diagnoseregeln Folgendes umfasst:

einen oder mehrere der extrahierten Bilddeskriptoren; und
ein individuelles Diagnoseergebnis (102), das dem einen bzw. den mehreren extrahierten Bilddeskriptoren entspricht; und

Steuern der Anzeige, um das abgeleitete umfassende Diagnoseergebnis zusammen mit der diagnostischen Grundlage anzuzeigen, die die Diagnoseregeln und die entsprechenden Bilddeskriptoren umfasst, welche zum Ableiten des umfassenden Diagnoseergebnisses verwendet wurden.

**2.** Medizinische Abbildungsdiagnosevorrichtung nach Anspruch 1, wobei der Prozessor weiterhin dazu konfiguriert ist, die Anzeige zu steuern, um das abgeleitete umfassende Diagnoseergebnis, die Diagnoseregeln oder eine Kombination davon basierend auf einem Zeichen, einem Bild, einer Farbe, einer Helligkeit, einer Luminanz oder einer beliebigen Kombination davon anzuzeigen; und/oder
wobei der Prozessor weiterhin dazu konfiguriert ist, die Anzeige zu steuern, um Folgendes anzuzeigen: die Genauigkeit des abgeleiteten umfassenden Diagnoseergebnisses, einen Wichtigkeitsgrad der Diagnoseregeln, die zum Ableiten des umfassenden Diagnoseergebnisses verwendet werden, die Genauigkeit der extrahierten Bilddeskriptoren, die in den Diagnoseregeln enthalten sind, die Genauigkeit des individuellen Diagnoseergebnisses, das in den Diagnoseregeln enthalten ist, oder eine beliebige Kombination davon basierend auf einem Zeichen, einem Bild, einer Farbe, einer Helligkeit, einer Luminanz oder einer beliebigen Kombination davon.

**3.** Medizinische Abbildungsdiagnosevorrichtung nach Anspruch 1 oder 2, wobei, wenn das abgeleitete umfassende Diagnoseergebnis keine widersprüchlichen Tatsachen aufweist, der Prozessor weiterhin dazu konfiguriert ist, die Diagnoseregeln mit den gleichen individuellen Diagnoseergebnissen zu gruppieren und die Anzeige dazu zu steuern, die umfassenden Diagnoseergebnisse durch Hervorheben anzuzeigen; und/oder
wenn das abgeleitete umfassende Diagnoseergebnis widersprüchliche Tatsachen aufweist, die Steuerung weiterhin dazu konfiguriert ist, die Diagnoseregeln mit unterschiedlichen individuellen Diagnoseergebnissen zu klassifizieren und die Anzeige dazu zu steuern, die widersprüchlichen Tatsachen individuell anzuzeigen.

**4.** Medizinische Abbildungsdiagnosevorrichtung nach einem der Ansprüche 1 bis 3, wobei der Prozessor weiterhin zu Folgendem konfiguriert ist:

Erstellen einer oder mehrerer der Diagnoseregeln unter Verwendung eines oder mehrerer medizinischer Referenzbilder; und
Zuweisen eines vorbestimmten Wichtigkeitsgrades zu jeder der erstellten Diagnoseregeln; und
wobei der Prozessor weiterhin dazu konfiguriert ist, den vorbestimmten Wichtigkeitsgrad basierend auf einer Unterstützung und einem Vertrauen jeder der erstellten Diagnoseregeln und einer Genauigkeit jedes der Bilddeskriptoren, die in den erstellten Diagnoseregeln enthalten sind, zu berechnen.

5. Medizinische Abbildungsdiagnosevorrichtung nach Anspruch 4, wobei der Prozessor weiterhin zu Folgendem konfiguriert ist:

   Auswählen einer oder mehrerer der Diagnoseregeln aus den erstellten Diagnoseregeln gemäß dem vorbestimmten Wichtigkeitsgrad der erstellten Diagnoseregeln; und
   Ableiten des umfassenden Diagnoseergebnisses für das medizinische Zielbild unter Verwendung der ausgewählten Diagnoseregeln.

6. Medizinische Abbildungsdiagnosevorrichtung nach einem der Ansprüche 1 bis 5, wobei der Prozessor weiterhin dazu konfiguriert ist, die eine oder mehreren Diagnoseregeln basierend auf Benutzerfeedback für das abgeleitete umfassende Diagnoseergebnis zu aktualisieren.

7. Medizinische Abbildungsdiagnosevorrichtung nach Anspruch 6, wobei der Prozessor weiterhin dazu konfiguriert ist, die Genauigkeit des abgeleiteten umfassenden Diagnoseergebnisses, einen Wichtigkeitsgrad der Diagnoseregeln, die zum Ableiten des umfassenden Diagnoseergebnisses verwendet werden, die Genauigkeit der extrahierten Bilddeskriptoren, die in den Diagnoseregeln enthalten sind, die Genauigkeit des individuellen Diagnoseergebnisses, das in den Diagnoseregeln enthalten ist, oder eine beliebige Kombination davon gemäß dem Benutzerfeedback zu aktualisieren.

8. Medizinische Abbildungsdiagnosevorrichtung nach Anspruch 6 oder 7, wobei der Prozessor weiterhin dazu konfiguriert ist, eine oder mehrere der Diagnoseregeln, einen oder mehrere der extrahierten Bilddeskriptoren, die in den Diagnoseregeln enthalten sind, oder eine beliebige Kombination davon gemäß dem Benutzerfeedback zu löschen.

9. Medizinische Abbildungsdiagnosevorrichtung nach einem der Ansprüche 6 bis 8, wobei, wenn das abgeleitete umfassende Diagnoseergebnis sich nach der Aktualisierung der einen bzw. mehreren Diagnoseregeln von einem umfassenden Diagnoseergebnis unterscheidet, der Prozessor weiterhin dazu konfiguriert ist, das abgeleitete umfassende Diagnoseergebnis, das umfassende Diagnoseergebnis nach der Aktualisierung der einen bzw. mehreren Diagnoseregeln, und Unterschiede zwischen dem abgeleiteten umfassenden Diagnoseergebnis und dem umfassenden Diagnoseergebnis nach der Aktualisierung der einen bzw. mehreren Diagnoseregeln anzuzeigen.

10. Medizinisches Abbildungsdiagnoseverfahren zum Bereitstellen einer diagnostischen Grundlage bei der Durchführung einer computergestützten Diagnose, um das Vorhandensein einer Läsion in einem medizinischen Zielbild zu diagnostizieren, wobei das Verfahren Folgendes umfasst:

    Extrahieren von charakteristischen Informationen, die mit einem Bereich in Verbindung stehen, von dem vermutet wird, dass es sich um eine Läsion in dem medizinischen Zielbild handelt, als ein oder mehrere Bilddeskriptoren aus dem medizinischen Zielbild;
    Ableiten eines umfassenden Diagnoseergebnisses für das medizinische Zielbild unter Verwendung einer oder mehrerer Diagnoseregeln, wobei jede der Diagnoseregeln einen oder mehrere der extrahierten Bilddeskriptoren und ein individuelles Diagnoseergebnis, das dem einen bzw. den mehreren extrahierten Bilddeskriptoren entspricht, enthält; und
    Anzeigen des abgeleiteten umfassenden Diagnoseergebnisses zusammen mit der diagnostischen Grundlage, die die Diagnoseregeln und die entsprechenden Bilddeskriptoren umfasst, welche zum Ableiten des umfassenden Diagnoseergebnisses verwendet wurden.

11. Medizinisches Abbildungsdiagnoseverfahren nach Anspruch 10, das weiterhin Folgendes umfasst:

    Erstellen einer oder mehrerer der Diagnoseregeln unter Verwendung eines oder mehrerer medizinischer Referenzbilder; und
    Zuweisen eines vorbestimmten Wichtigkeitsgrades zu jeder der erstellten Diagnoseregeln.

12. Medizinisches Abbildungsdiagnoseverfahren nach Anspruch 10, das weiterhin ein Aktualisieren der einen oder mehreren Diagnoseregeln basierend auf Benutzerfeedback für das abgeleitete umfassende Diagnoseergebnis umfasst.

**Revendications**

1. Appareil de diagnostic par imagerie médicale (200) permettant de fournir une base de diagnostic (402, 502, 602, 702) lors de la réalisation d'un diagnostic assisté par ordinateur pour diagnostiquer la présence d'une lésion sur une image médicale cible, l'appareil comprenant :

    un écran,
    une mémoire conçue pour contenir des instructions, et
    un processeur conçu pour, lors de l'exécution des instructions :

        extraire, à partir de l'image médicale cible (120), des informations caractéristiques en lien avec une zone suspectée d'être une lésion sur l'image médicale cible et servant de descripteur(s) d'image (101) ;
        dériver un résultat diagnostique global (401, 501, 601, 701) relativement à l'image médicale cible au moyen d'une ou plusieurs règles de diagnostic (100, 402a, 402b, 402c, 502a, 502b, 602a, 602b, 602c, 702a, 702b), chacune des règles de diagnostic comprenant :

            un ou plusieurs des descripteurs d'image extraits, et
            un résultat diagnostique individuel (102) correspondant aux un ou plusieurs descripteurs d'image extraits ; et

        commander à l'écran d'afficher le résultat diagnostique global dérivé, conjointement à la base de diagnostic comprenant les règles de diagnostic et les descripteurs d'image correspondants servant à dériver le résultat diagnostique global.

2. Appareil de diagnostic par imagerie médicale selon la revendication 1, dans lequel le processeur est conçu en outre pour commander à l'écran d'afficher le résultat diagnostique global dérivé, les règles de diagnostic ou une combinaison de ceux-ci compte tenu d'un caractère, d'une image, d'une couleur, de la luminosité, de la luminance ou de toute combinaison de ceux-ci ; et/ou dans lequel le processeur est conçu en outre pour commander à l'écran d'afficher l'exactitude du résultat diagnostique global dérivé, un degré d'importance des règles de diagnostic servant à dériver le résultat diagnostique global, l'exactitude des descripteurs d'image extraits présents dans les règles de diagnostic, l'exactitude du résultat diagnostique individuel présent dans les règles de diagnostic ou toute combinaison de ceux-ci compte tenu d'un caractère, d'une image, d'une couleur, de la luminosité, de la luminance ou de toute combinaison de ceux-ci.

3. Appareil de diagnostic par imagerie médicale selon la revendication 1 ou 2, dans lequel, lorsque le résultat diagnostique global dérivé ne contient aucun élément contradictoire, le processeur est conçu en outre pour grouper les règles de diagnostic présentant les mêmes résultats diagnostiques individuels et pour commander à l'écran d'afficher les résultats diagnostiques globaux par mise en surbrillance, et/ou
lorsque le résultat diagnostique global dérivé contient des éléments contradictoires, le processeur est conçu en outre pour catégoriser les règles de diagnostic présentant des résultats diagnostiques individuels différents et pour commander à l'écran d'afficher les éléments contradictoires individuellement.

4. Appareil de diagnostic par imagerie médicale selon l'une quelconque des revendications 1 à 3, dans lequel le processeur est conçu en outre pour :

    créer une ou plusieurs des règles de diagnostic au moyen d'une ou plusieurs images médicales de référence, et attribuer un degré d'importance prédéterminé à chacune des règles de diagnostic créées ;
    le processeur étant conçu en outre pour calculer le degré d'importance prédéterminé, compte tenu d'une confirmation et d'une confiance relatives à chacune des règles de diagnostic créées, et l'exactitude de chacun des descripteurs d'image présents dans les règles de diagnostic créées.

5. Appareil de diagnostic par imagerie médicale selon la revendication 4, dans lequel le processeur est conçu en outre pour :

    sélectionner une ou plusieurs des règles de diagnostic parmi les règles de diagnostic créées, conformément au degré d'importance prédéterminé des règles de diagnostic créées, et
    dériver le résultat diagnostique global relativement à l'image médicale cible au moyen des règles de diagnostic sélectionnées.

**6.** Appareil de diagnostic par imagerie médicale selon l'une quelconque des revendications 1 à 5, dans lequel le processeur est conçu en outre pour actualiser les une ou plusieurs règles de diagnostic compte tenu d'une rétroaction de la part de l'utilisateur eu égard au résultat diagnostique global dérivé.

**7.** Appareil de diagnostic par imagerie médicale selon la revendication 6, dans lequel le processeur est conçu en outre pour actualiser l'exactitude du résultat diagnostique global dérivé, le degré d'importance des règles de diagnostic servant à dériver le résultat diagnostique global, l'exactitude des descripteurs d'image extraits présents dans les règles de diagnostic, l'exactitude du résultat diagnostique individuel présent dans les règles de diagnostic ou toute combinaison de ceux-ci conformément à la rétroaction de la part de l'utilisateur.

**8.** Appareil de diagnostic par imagerie médicale selon la revendication 6 ou 7, dans lequel le processeur est conçu en outre pour supprimer une ou plusieurs des règles de diagnostic, un ou plusieurs des descripteurs d'image extraits présents dans les règles de diagnostic, ou une combinaison de ceux-ci, conformément à la rétroaction de la part de l'utilisateur.

**9.** Appareil de diagnostic par imagerie médicale selon l'une quelconque des revendications 6 à 8, dans lequel, lorsque le résultat diagnostique global dérivé est différent d'un résultat diagnostique global après actualisation des une ou plusieurs règles de diagnostic, le processeur est conçu en outre pour afficher le résultat diagnostique global dérivé, le résultat diagnostique global après actualisation des une ou plusieurs règles de diagnostic, et les différences entre le résultat diagnostique global dérivé et le résultat diagnostique global après actualisation des une ou plusieurs règles de diagnostic.

**10.** Procédé de diagnostic par imagerie médicale visant à fournir une base de diagnostic lors de la réalisation d'un diagnostic assisté par ordinateur pour diagnostiquer la présence d'une lésion sur une image médicale cible, le procédé comprenant :

l'extraction, à partir de l'image médicale cible, d'informations caractéristiques en lien avec une zone suspectée d'être une lésion sur l'image médicale cible et servant de descripteur(s) d'image, la dérivation d'un résultat diagnostique global relativement à l'image médicale cible au moyen d'une ou plusieurs règles de diagnostic, chacune des règles de diagnostic comprenant un ou plusieurs des descripteurs d'image extraits et un résultat diagnostique individuel correspondant aux un ou plusieurs descripteurs d'image extraits, et
l'affichage du résultat diagnostique global dérivé, conjointement à la base de diagnostic comprenant les règles de diagnostic et les descripteurs d'image correspondants servant à dériver le résultat diagnostique global.

**11.** Procédé de diagnostic par imagerie médicale selon la revendication 10, comprenant en outre :

la création d'une ou plusieurs règles de diagnostic au moyen d'une ou plusieurs images médicales de référence, et
l'attribution d'un degré d'importance prédéterminé à chacune des règles de diagnostic créées.

**12.** Procédé de diagnostic par imagerie médicale selon la revendication 10, comprenant en outre l'actualisation d'une ou plusieurs règles de diagnostic compte tenu d'une rétroaction de la part de l'utilisateur eu égard au résultat diagnostique global dérivé.

EP 2 608 152 B1

# FIG. 1

IMAGE DESCRIPTOR

INDIVIDUAL DIAGNOSTIC RESULT

# FIG. 2

# FIG. 3

| DIAGNOSTIC RULE | DEGREE OF IMPORTANCE |
|---|---|
| Rule 1 : [ ( x1, y1 ) $\longrightarrow$ M ] | 98 |
| Rule 2 : [ ( x2, y2 ) $\longrightarrow$ B ] | 95 |
| Rule 3 : [ ( x1, z1 ) $\longrightarrow$ M ] | 90 |
| Rule 4 : [ ( x2, z2 ) $\longrightarrow$ M ] | 80 |
| Rule 5 : [ ( x3, z3 ) $\longrightarrow$ B ] | 98 |
| Rule 6 : [ ( x1, z3 ) $\longrightarrow$ B ] | 40 |
| Rule 7 : [ ( x1, y3 ) $\longrightarrow$ B ] | 75 |
| ⋮ | ⋮ |

## FIG. 4

400

**402a** → Rule 1 (DEGREE OF IMPORTANCE OF 98)
- Image Descriptor X 95%
- Image Descriptor Y 89%
- ⇒ Malignant 98%

**402** { **402b** → Rule 2 (DEGREE OF IMPORTANCE OF 94)
- Image Descriptor X 95%
- Image Descriptor Z 85%
- ⇒ Malignant 97%

**402c** → Rule 3 (DEGREE OF IMPORTANCE OF 85)
- Image Descriptor Y 89%
- Image Descriptor Z 85%
- ⇒ Malignant 90%

Malignancy

401

EP 2 608 152 B1

# FIG. 5

EP 2 608 152 B1

## FIG. 6

600

Rule 1 (DEGREE OF IMPORTANCE OF 98)

602a

| Image Descriptor X 95% | Image Descriptor Y 89% | ⇨ | Malignant 98% |

Rule 2 (DEGREE OF IMPORTANCE OF 94)

602b

| Image Descriptor X 95% | Image Descriptor Z 85% | ⇨ | Malignant 97% |

Rule 3 (DEGREE OF IMPORTANCE OF 85)

602c

| Image Descriptor W 89% | Image Descriptor Z 83% | ⇨ | Benign 90% |

Malignancy 97.5%

601a

601

Benign 90%

601b

EP 2 608 152 B1

FIG. 7

700

701a

701

701b

Original : Malignancy 98%

User Defined: Malignancy 97%

Rule 1 (DEGREE OF IMPORTANCE OF 99) : Original Setting

Image Descriptor X 95%

Image Descriptor Y 89%

Malignant 98%

Rule 1 (DEGREE OF IMPORTANCE OF 94) : User defined Setting

Image Descriptor X 95%

Image Descriptor Z 85%

Malignant 97%

702a

702

702b

# FIG. 8A

| Rule 2 (DEGREE OF IMPORTANCE OF 95) | | |
|---|---|---|
| Image Descriptor X 95% | Image Descriptor Z 85% | ⇨ Malignant 97% |

➡

| Rule 2 (DEGREE OF IMPORTANCE OF 96) | | |
|---|---|---|
| Image Descriptor X 90% | Image Descriptor Z 99% | ⇨ Malignant 97% |

# FIG. 8B

EP 2 608 152 B1

| Rule 1 (DEGREE OF IMPORTANCE OF 98) | | |
|---|---|---|
| Image Descriptor X 95% | Image Descriptor Y 89% | ⇨ Malignant 98% |

➡

| Rule 1 (DEGREE OF IMPORTANCE OF 98) | | |
|---|---|---|
| Image Descriptor X 95% | Image Descriptor Y 89% | ⇨ Malignant 98% |

| Rule 2 (DEGREE OF IMPORTANCE OF 95) | | |
|---|---|---|
| Image Descriptor X 95% | Image Descriptor Z 85% | ⇨ Malignant 97% |

FIG. 8C

EP 2 608 152 B1

Rule 1 (DEGREE OF IMPORTANCE OF 98)

| Image Descriptor X 95% | Image Descriptor Y 89% | ⇨ | Malignant 98% |

➡

Rule 1 (DEGREE OF IMPORTANCE OF 98)

| Image Descriptor X 95% | ⇨ | Malignant 98% |

# FIG. 9

START

CREATE DIAGNOSTIC RULE ~ 901

ASSIGN DEGREE OF IMPORTANCE ~ 902

EXTRACT IMAGE DESCRIPTOR
FROM TARGET MEDICAL IMAGE ~ 903

DERIVE DIAGNOSTIC RESULT
USING DIAGNOSTIC RULE ~ 904

DISPLAY DIAGNOSTIC RESULT
TOGETHER WITH DIAGNOSTIC BASIS ~ 905

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007255512 A